(19) Europäisches Patentamt
European Patent Office
Office européen des brevets

(11) Veröffentlichungsnummer: **0 192 117**
**B1**

(12) # EUROPÄISCHE PATENTSCHRIFT

(45) Veröffentlichungstag der Patentschrift: **26.09.90**

(21) Anmeldenummer: **86101406.6**

(22) Anmeldetag: **04.02.86**

(51) Int. Cl.$^5$: **C 07 D 285/12,**
C 07 D 417/12, A 01 N 43/82

(54) **5-Chlor-1,3,4-thiadiazol-2-yloxy-acetamide.**

(30) Priorität: **16.02.85 DE 3505425**

(43) Veröffentlichungstag der Anmeldung:
**27.08.86 Patentblatt 86/35**

(45) Bekanntmachung des Hinweises auf die Patenterteilung:
**26.09.90 Patentblatt 90/39**

(84) Benannte Vertragsstaaten:
**AT BE CH DE FR GB IT LI NL**

(56) Entgegenhaltungen:
**EP-A-0 018 497**
**EP-A-0 029 171**
**EP-A-0 094 541**

(73) Patentinhaber: **BAYER AG**
**D-5090 Leverkusen 1 Bayerwerk (DE)**

(72) Erfinder: **Förster, Heinz, Dr.**
**Am Eckbuch 47**
**D-5600 Wuppertal 1 (DE)**
Erfinder: **Santel, Hans-Joachim, Dr.**
**Grünstrasse 9a**
**D-5090 Leverkusen (DE)**
Erfinder: **Schmidt, Robert R., Dr.**
**Im Waldwinkel 110**
**D-5060 Bergisch-Gladbach 2 (DE)**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 192 117 B1

**Beschreibung**

Die Erfindung betrifft neue 5-Chlor-1,3,4-thiadiazol-2-yloxy-acetamide, ein Verfahren zur ihrer Herstellung und ihre Verwendung als Herbizide.

Es ist bereits bekannt, daß bestimmte Heteroaryloxyacetamide, wie beispielsweise das 2-(Benzthiazol-2-yloxy)-N-methylacetanilid, herbizide Eigenschaften besitzen (vgl. z.B. DE—OS 28 22 155, DE—OS 29 03 966 bzw. EP—A 5501). Die herbizide Wirksamkeit dieser vorbekannten Heteroaryloxyacetamide gegenüber Schadpflanzen ist jedoch nicht immer in allen Anwendungsbereichen völlig zufriedenstellend.

Es wurden neue 5-Chlor-1,3,4-thiadiazol-2-yloxy-acetamide der allgemeinen Formel (I) gefunden,

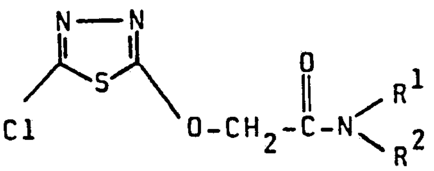

(I)

in welcher

$R^1$ und $R^2$ unabhängig voneinander für geradkettiges oder verzweigtes Alkyl mit 1 bis 8 Kohlenstoffatomen, geradkettiges oder verzweigtes Alkenyl und Alkinyl mit jeweils 2 bis 8 Kohlenstoffatomen, für gegebenenfalls einfach oder mehrfach, gleich oder verschieden substituiertes Cycloalkyl oder Cycloalkenyl mit jeweils 3 bis 7 Kohlenstoffatom, wobei als Substituenten insbesondere Alkylreste mit 1 bis 4 Kohlenstoffatomen in Frage kommen, für jeweils geradkettiges oder verzweigtes Alkoxy, Alkoxyalkylenoxy oder Alkoxyalkyl mit jeweils 1 bis 8 Kohlenstoffatomen in den einzelnen Alkyl- bzw. Alkylenteilen, für Halogenalkyl mit 1 bis 8 Kohlenstoffatomen und 1 bis 5 Halogenatomen (insbesondere Fluor, Chlor und Brom), für Aralkyl mit 6 bis 10 Kohlenstoffatomen im Arylteil und 1 bis 2 Kohlenstoffatomen im Alkylteil, sowie für gegebenenfalls einfach oder mehrfach, gleich oder verschieden substituiertes Aryl mit 6 bis 10 Kohlenstoffatomen stehen, wobei als Substituenten in Frage kommen: Halogen, geradkettiges oder verzweigtes Alkyl, Alkoxy oder Alkylthio mit jeweils 1 bis 4 Kohlenstoffatomen, Halogenalkyl, Halogenalkoxy und Halogenalkylthio mit jeweils 1 bis 2 Kohlenstoffatomen und 1 bis 5 Halogenatomen (insbesondere Fluor, Chlor und Brom), sowie Nitro, oder

$R^1$ und $R^2$ zusammen mit dem Stickstoffatom, an das sie gebunden sind, für einen gegebenenfalls einfach oder mehrfach, gleich oder verschieden substituierten, gesättigten oder ungesättigten, 5- bis 7-gliedrigen Heterocyclus stehen (wobei als Substituenten in Frage kommen: geradkettiges oder verzweigtes Alkyl mit 1 bis 6 Kohlenstoffatomen, auch in Form eines anellierten Ringsystems, Aryl mit 6 bis 10 Kohlenstoffatomen, auch in Form eines anellierten Ringsystems oder Dioxyalkylen mit 2 bis 3 Kohlenstoffatomen).

Weiterhin wurde gefunden, daß man die neuen 5-Chlor-1,3,4-thiadiazol-2-yloxy-acetamide der allgemeinen Formel (I) erhält, wenn man 2-Alkylsulfonyl-5-chlor-1,3,4-thiadiazole der Formel (II)

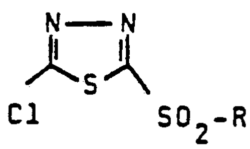

(II)

in welcher

R für Alkyl steht,

mit Glykolsäureamiden der Formel (III),

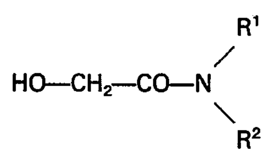

(III)

in welcher

$R^1$ und $R^2$ die oben angegebene Bedeutung haben,

gegebenenfalls in Gegenwart eines Verdünnungsmittels und gegebenenfalls in Gegenwart eines Säurebindemittels sowie eines Katalysators umsetzt.

Schließlich wurde gefunden, daß die neuen 5-Chlor-1,3,4-thiadiazol-2-yloxy-acetamide der allgemeinen Formel (I) herbizide, insbesondere auch selektiv-herbizide Eigenschaften besitzen.

Überraschenderweise zeigen die neuen 5-Chlor-1,3,4-thiadiazol-2-yloxy-acetamide der Formel (I) eine erheblich verbesserte herbizide Wirksamkeit gegen verbreitete, schwer bekämpfbare Unkräuter bei vergleichbar hoher Verträglichkeit gegenüber wichtigen Kulturpflanzen im Vergleich zu den aus dem Stand

der Technik vorbekannten Heteroaryloxyacetamiden, wie beispielsweise dem 2-(Benzthiazol-2-yloxy)-N-methyl-acetanilid, welche chemisch und wirkungsmäßig naheliegende Verbindungen sind.

Die erfindungsgemäßen 5-Chlor-1,3,4-thiadiazol-2-yloxy-acetamide sind durch die Formel (I) allgemein definiert. Bevorzugt sind Verbindungen der Formel (I), bei welchen

$R^1$ und $R^2$ unabhängig voneinander für geradkettiges oder verzweigtes Alkyl mit 1 bis 6 Kohlenstoffatomen, für geradkettiges oder verzweigtes Alkenyl und Alkinyl mit jeweils 2 bis 6 Kohlenstoffatomen, für gegebenenfalls ein- bis dreifach, gleich oder verschieden durch Methyl oder Ethyl substituiertes Cycloalkyl bzw. Cycloalkenyl mit 5 bis 7 Kohlenstoffatomen, für jeweils geradkettiges oder verzweigtes Alkoxy, Alkoxyalkylenoxy oder Alkoxyalkyl mit jeweils 1 bis 6 Kohlenstoffatomen in den einzelnen Alkylteilen, für Halogenalkyl mit 1 bis 6 Kohlenstoffatomen und 1 bis 5 Halogenatomen (insbesondere Fluor, Brom und Chlor), für Benzyl sowie für gegebenenfalls ein- bis dreifach, gleich oder verschieden substituiertes Phenyl stehen, wobei als Substituenten besonders bevorzugt sind: Methyl, Ethyl, Methoxy, Methylthio, Trifluormethyl, Trifluormethoxy, Trifluormethylthio, Fluor, Chlor oder Nitro; oder

$R^1$ und $R^2$ zusammen mit dem Stickstoffatom, an das sie gebunden sind, für einen gegebenenfalls einbis dreifach, gleich oder verschieden substituierten Heterocyclus der Formel

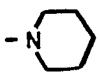 ; 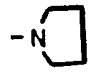 ; 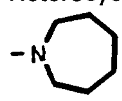 ; 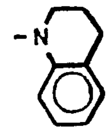

stehen, wobei als Substituenten besonders bevorzugt sind: Methyl, Ethyl und Phenyl.

Im einzelnen seien außer den bei den Herstellungsbeispielen genannten Verbindungen die folgenden Verbindungen der allgemeinen Formel (I) genannt:

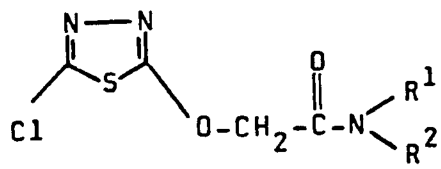

(I)

Tabelle 1

| $R^1$ | $R^2$ | $R^1$ | $R^2$ |
|---|---|---|---|
| $(CH_3)_2CH-O-$ | $CH_3$ | 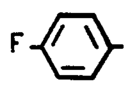 | $CH_3$ |
| $CH_3O-$ | $C_2H_5$ | 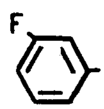 | $CH_3$ |
| 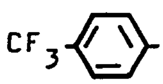 | $CH_3$ | $-CH_2-CH_2-O-CH_2-CH_2-$ | |
| 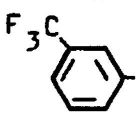 | $CH_3$ | 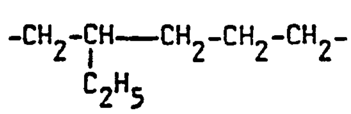 | |
| 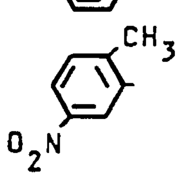 | $CH_3$ | 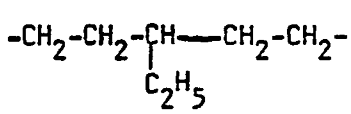 | |
| 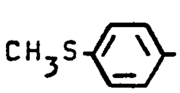 | $CH_3$ | $CH_3OCH_2-$ | $CH_3$ |
| 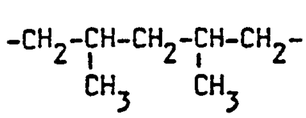 | | $F_3C-CH_2-$ | $CH_3$ |
| 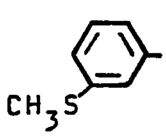 | $CH_3$ | 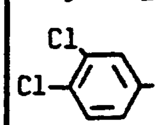 | $CH_3$ |

Verwendet man beispielsweise 5-Chlor-3-methylsulfonyl-1,3,4-thiadiazol und Glykolsäure-N-methylanilid als Ausgangsstoffe, so läßt sich der Reaktionsablauf des erfindungsgemäßen Verfahrens durch das folgende Formelschema darstellen:

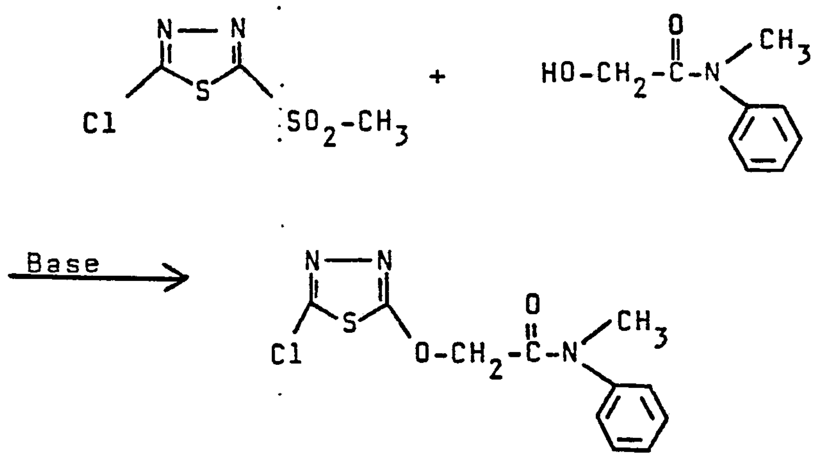


Die zur Durchführung des erfindungsgemäßen Verfahrens als Ausgangsstoffe benötigten 2-Alkylsulfonyl-5-chlor-1,3,4-thiadiazole sind durch die Formel (II) allgemeinen definiert. Bevorzugt sind Verbindungen der Formel (II) bei welchen R für geradkettiges oder verzweigtes Alkyl mit 1 bis 5 Kohlenstoffatomen, insbesondere für Methyl oder Ethyl steht. Die 2-Alkylsulfonyl-5-chlor-1,3,4-thiadiazole der Formel (II) sind bekannt (vgl. z.B. DE—OS 21 44 326).

Die zur Durchführung des erfindungsgemäßen Verfahrens weiterhin als Ausgangsstoffe benötigten Glykolsäureamide sind durch die Formel (III) allgemein definiert. In dieser Formel (III) stehen $R^1$ und $R^2$ vorzugsweise für diejenigen Reste, die schon bei der Beschreibung der erfindungsgemäßen Stoffe der Formel (I) als bevorzugt für diese Substituenten genannt wurden. Die Glykolsäureamide der Formel (III) sind ebenfalls bekannt (vgl. z.B. DE—OS 29 04 490, EP—OS 5501, EP—OS 29 171, DE—OS 30 38 598, DE—OS 32 44 956).

Als Verdünnungsmittel für das erfindungsgemäße Verfahren kommen organische oder anorganische Lösungsmittel in Frage. Bevorzugt sind Kohlenwasserstoffe, wie Toluol oder Cyclohexan, Halogenkohlenwasserstoffe, wie Methylenchlorid, Chloroform, Dichlorethan oder Chlorbenzol, Ketone, wie Aceton oder Methylisobutylketon, Ether, wie Diethylether, Diisopropylether oder Methyl-t-butylether, Alkohole, wie Methanol, Ethanol oder Isopropanol, Amide, wie Dimethylformamid oder Dimethylacetamid, Sulfoxide, wie Dimethylsulfoxid, Wasser oder wäßrige Salzlösungen.

Als Salze verwendet man hierbei vorzugsweise Chloride oder Sulfate von Alkali- oder Erdalkalimetallen, wie beispielsweise Natriumchlorid, Kaliumchlorid oder Calciumchlorid. Besonders bevorzugt ist Natriumchlorid.

Das erfindungsgemäße Verfahren wird vorteilhaft unter Verwendung von Säurebindemitteln durchgeführt. Als solche werden vorzugsweise stark basische Alkali- und Erdalkalimetallverbindungen, beispielsweise Oxide, wie z.B. Natrium-, Kalium-, Magnesium- und Calciumoxid, Hydroxide, wie z.B. Natrium-, Kalium-, Magnesium- und Calciumhydroxid und/oder Carbonate, wie z.B. Natrium-, Kalium-, Magnesium- und Calciumcarbonat verwendet.

Der Zusatz von 0,01 bis 10 Gew.-% (bezogen auf eingesetztes Glykolsäureamid der Formel (III)) eines Phasentransferkatalysators mag sich in einigen Fällen als vorteilhaft erweisen. Als Beispiele für solche Katalysatoren seien genannt:

Tetrabutylammoniumchlorid, Tetrabutylammoniumbromid, Tributyl-methylphosphoniumbromid, Trimethyl-$C_{13}$/$C_{15}$-alkyl-ammoniumchlorid, Dibenzyl-dimethyl-ammonium-methylsulfat, Dimethyl-$C_{12}$/$C_{14}$-alkyl-benzylammoniumchlorid, Tetrabutylammoniumhydroxid, 18-Krone-6, Triethylbenzylammoniumchlorid, Trimethylbenzylammoniumchlorid, Tetraethylammoniumbromid.

Die Reaktionstemperaturen können bei dem erfindungsgemäßen Verfahren innerhalb eines größeren Bereiches variiert werden. Sie liegen im allgemeinen zwischen −50°C und +100°C, vorzugsweise zwischen −20°C und +100°C.

Das erfindungsgemäße Verfahren wird im allgemeinen bei Normaldruck durchgeführt, es kann aber auch bei erhöhtem oder vermindertem Druck, etwa zwischen 0,1 und 10 bar, durchgeführt werden.

Zur Durchführung des erfindungsgemäßen Verfahrens setzt man pro Mol 2-Alkylsulfonyl-5-chlor-1,3,4-thiadiazol der Formel (II) im allgemeinen 0,1 bis 10 Mol, vorzugsweise 0,8 bis 1,2 Mol an Glykolsäureamid der Formel (III) und 0,5 bis 10 Mol, vorzugsweise 0,5 bis 3 Mol an Base ein.

Die Zugabereihenfolge der Reaktanten kann beliebig vertauscht werden, es können auch alle Komponenten gleichzeitig in das Reaktionsgefäß eindosiert werden. Die Reaktionsführung kann

kontinuierlich oder diskontinuierlich gestaltet werden. Die Aufarbeitung erfolgt in überlicher Art und Weise.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzuen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden.

*Dikotyle Unkräuter der Gattungen:* Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

*Dikotyle Kulturen der Gattungen:* Gossypium, Glycine, Beta, Daucus, Phaseolus, Pisum, Solanum, Linum, Ipomoea, Vicia, Nicatiana, Lycopersicon, Arachis, Brassica, Lactuca, Cucumis, Cucurbita.

*Monokotyle Unkräuter der Gattungen:* Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

*Monokotyle Kulturen der Gattungen:* Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung ein einjährigen Kulturen eingesetzt werden.

Dabei zeigen die erfindungsgemäßen Wirkstoffe neben einer hervorragenden Wirkung gegenüber Schadpflanzen auch eine gute Verträglichkeit gegenüber wichtigen Kulturpflanzen und können daher als selektive Unkrautbekämpfungsmittel in monokotylen Kulturen, wie Getreide und Reis ebenso wie in dikotylen Kulturen, wie Sojabohnen, Baumwolle, Zuckerrüben und anderen eingesetzt werden.

Darüber hinaus weisen die erfindungsgemäßen Wirkstoffe in entsprechenden Aufwandmengen auch eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Reiskrankheiten wie beispielsweise gegen den Erreger der Reisfleckenkrankheit (Pyricularia oryzae) einsetzen.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittels als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/

oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, naturliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive konnen mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe, und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4-(1H,3H)-dion oder N-(2-Benzthiazolyl)-N,N'-dimethylharnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1-,1-dimethylethyl)-3-methylthio-1,2,4-triazin-5(4H)-on zur Unkrautbekämpfung in Sojabohnen, in Frage. Auch Mischungen mit N,N'-Dimethyl-N'-(3-trifluormethylphenyl)-harnstoff, N,N-Dimethyl-N'-(3-chlor-4-methylphenyl)-harnstoff, N,N-Dimethyl-N'-(4-isopropylphenyl)-harnstoff, 4-Amino-6-t-butyl-3-ethylthio-1,2,4-triazin-5(4H)-on, 2,4-Dichlorphenoxyessigsäure, 2,4-Dichlorphenoxypropionsäure, (2-Methyl-4-chlorphenoxy)-essigsäure, (4-Chlor-2-methylphenoxy)-propionsäure, 2-[4-(3,5-Dichlorpyrid-2-yloxy)-phenoxy]-propionsäure-(2-benzyloxy-ethylester), -(trimethylsilylmethylester) oder -(2,2-diethoxy-ethylester), Propionsäure-N-(3,4-dichlorphenyl)-amid; 2-Chlor-4-N-ethylamino-6-N-isopropylamino-1,3,5-triazin; N-Methyl-2-(1,3-benzthiazol-2-yloxy)-acetanilid und 2-{4-[[3-Chlor-5-(trifluormethyl)-2-pyrimidinyl]-oxy]-phenoxy}-propanesäure bzw. -propansäureethylester sind möglich. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen bei der Anwendung als Herbizide zwischen 0,01 und 10 kg Wirkstoff pro Hektar Bodenfläche, vorzugsweise zwischen 0,05 und 5 kg pro ha.

Die nachfolgenden Beispiele dienen zur weiteren Erläuterung der Erfindung.

Herstellungsbeispiele:

Beispiel 1

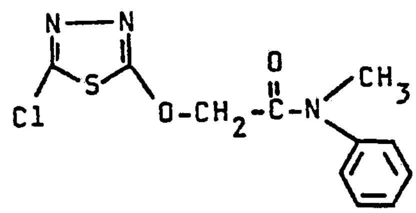

Zu einer Mischung aus 8,3 g (0,05 Mol) Glykolsäure-N-methylanilid und 3,1 g (0,05 Mol) pulverisiertem Kaliumhydroxid in 100 ml Isopropanol gibt man bei −20°C eine Lösung von 10,6 g (0,05 Mol) 2-Chlor-5-ethylsulfonyl-1,3,4-thiadiazol in 30 ml Acetonitril und rührt nach beendeter Zugabe eine Stunde bei −15°C nach. Zur Aufarbeitung gießt man die Reaktionsmischung in Wasser, reibt das ausfallende Öl kristallin, saugt den Feststoff ab, wäscht mit Wasser und Ligroin nach und trocknet im Vakuum.

Man erhält 10 g (75% der Theorie) an 2-(5-Chlor-1,3,4-thiadiazol-2-yloxy)-N-methyl-acetanilid vom Schmelzpunkt 101°C.

In entsprechender Weise und gemäß den allgemeinen Angaben zur Herstellung erhält man die folgenden 5-Chlor-1,3,4-thiadiazol-2-yloxy-acetamide der allgemeinen Formel (I):

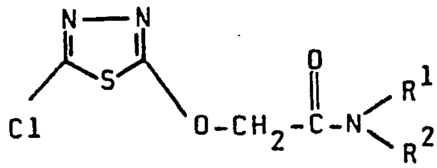 (I)

Tabelle 2

| Beispiel Nr. | $R^1$ | $R^2$ | physikalische Daten |
|---|---|---|---|
| 2 | $CH_3-(CH_2)_2-$ | $CH_3-(CH_2)_2-$ | $n_D^{20}$ 1,5230 |
| 3 | $CH_3O-$ | $CH_3$<br>\|<br>$C_2H_5-CH-$ | $n_D^{20}$ 1,5142 |
| 4 | $-CH_2-CH_2-CH_2-CH_2-CH_2-CH_2-$ | | Fp 91°C |
| 5 | $CH_3$ | $CH_3$ | |
| 6 | $(CH_3)_2CH-$ | $C_2H_5-O-CH_2-CH_2-O-$ | $n_D^{20}$ 1.5002 |
| 7 | $CH_3$ | 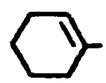 | Fp 95°C |
| 8 | $CH_3-(CH_2)_3-$ | $CH_3-(CH_2)_3-$ | $n_D^{20}$ 1,5005 |
| 9 | $CH_3$<br>\|<br>$-CH-CH_2-CH_2-CH_2-CH_2-$ | | $n_D^{20}$ 1,5409 |
| 10 | $C_2H_5$<br>\|<br>$-CH-CH_2-CH_2-CH_2-CH_2-$ | | $n_D^{20}$ 1,5403 |
| 11 | $CH_3$<br>\|<br>$-CH_2-CH_2-CH-CH_2-CH_2-$ | | Fp 88°C |
| 12 | $CH_3$ | $CH_3$<br>\|<br>$C_2H_5-CH-$ | $n_D^{20}$ 1,5215 |
| 13 | $CH_3$ $CH_3$<br>\| \|<br>$-CH-CH_2-CH-CH_2-CH_2-$ | | $n_D^{20}$ 1,5328 |
| 14 | 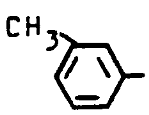 | $CH_3$ | Fp 76°C |

Tabelle 2

| Beispiel Nr. | $R^1$ | $R^2$ | physikalische Daten |
|---|---|---|---|
| 15 | $CH_3$ | $HC\equiv C-CH_2-$ | Fp 81°C |
| 16 | $CH_3$ | 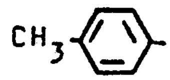 | Fp 109°C |
| 17 | $-CH_2-CH_2-CH_2-$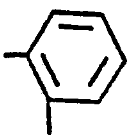 | | Fp 80°C |
| 18 | $C_2H_5$ | $C_2H_5$ | $n_D^{20}$ 1,5259 |
| 19 | $CH_3$ | 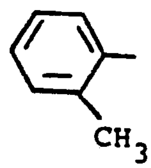 | Fp 112-113°C |
| 20 | $CH_3$ | 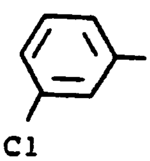 | Fp 94-98°C |

Anwendungsbeispiele:

In den folgenden Anwendungsbeispielen wurde die nachstehend aufgeführte Verbindung als Vergleichssubstanz herangezogen:

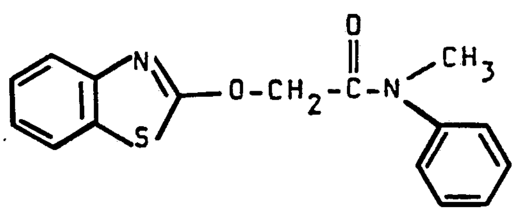 (A)

2-(Benzthiazol-2-yloxy)-N-methyl-acetanilid (bekannt aus DE—OS 2 903 966 bzw. EP 5501).

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)
100% = totale Vernichtung

Eine deutliche Uberlegenheit in der Wirksamkeit bei vergleichbarer Nutzpflanzenselektivität gegenüber dem Stand der Technik zeigen in diesem Test z.B. die Verbindungen gemäß folgender Herstellungsbeispiele: 1, 2, 4, 7, 14, 15, 17.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5—15 cm haben so, daß die jeweils gewünschten Wirkstoffmengen pro Flächeneinheit ausgebracht werden. Die Konzentration der Spritzbrühe wird so gewählt, daß in 2000 1 Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)
100% = totale Vernichtung

Eine deutliche Uberlegenheit in der Wirksamkeit bei vergleichbar Nutzpflanzenselektivität gegenüber dem Stand der Technik zeigen in diesem Test z.B. die Verbindungen gemäß folgender Herstellungsbeispiele: 1 und 2.

**Patentansprüche**

1. 5-Chlor-1,3,4-thiadiazol-2-yloxy-acetamide der allgemeinen Formel (I)

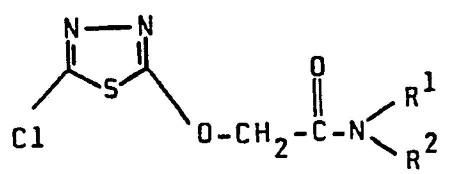

(I)

in welcher

$R^1$ und $R^2$ unabhängig voneinander für geradkettiges oder verzweigtes Alkyl mit 1 bis 8 Kohlenstoffatomen, geradkettiges oder verzweigtes Alkenyl und Alkinyl mit jeweils 2 bis 8 Kohlenstoffatomen, für gegebenenfalls einfach oder mehrfach, gleich oder verschieden substituiertes Cycloalkyl oder Cycloalkenyl mit jeweils 3 bis 7 Kohlenstoffatomen (wobei als Substituenten insbesondere Alkylreste mit 1 bis 4 Kohlenstoffatomen in Frage kommen), für jeweils geradkettiges oder verzweigtes Alkoxy, Alkoxyalkylenoxy oder Alkoxyalkyl mit jeweils 1 bis 8 Kohlenstoffatomen in den einzelnen Alkyl- bzw. Alkylenteilen, für Halogenalkyl mit 1 bis 8 Kohlenstoffatomen und 1 bis 5 Halogenatomen, für Aralkyl mit 6 bis 10 Kohlenstoffatomen im Arylteil und 1 bis 2 Kohlenstoffatomen im Alkylteil, sowie für gegebenenfalls einfach oder mehrfach, gleich oder verschieden substituiertes Aryl mit 6 bis 10 Kohlenstoffatomen stehen (wobei als Substituenten in Frage kommen: Halogen, geradkettiges oder verzweigtes Alkyl, Alkoxy oder Alkylthio mit jeweils 1 bis 4 Kohlenstoffatomen, Halogenalkyl, Halogenalkoxy und Halogenalkylthio mit jeweils 1 bis 2 Kohlenstoffatomen und 1 bis 5 Halogenatomen sowie Nitro), oder

$R^1$ und $R^2$ zusammen mit dem Stickstoffatom, an das sie gebunden sind, für einen gegebenenfalls einfach oder mehrfach, gleich oder verschieden substituierten, gesättigten oder ungesättigten, 5- bis 7-gliedrigen Heterocyclus stehen (wobei als Substituenten in Frage kommen: geradkettiges oder verzweigtes Alkyl mit 1 bis 6 Kohlenstoffatomen, auch in Form eines anellierten Ringsystems, Aryl mit 6 bis 10 Kohlenstoffatomen, auch in Form eines anellierten Ringsystems oder Dioxyalkylen mit 2 bis 3 Kohlenstoffatomen).

2. Verbindungen der allgemeinen Formel (I) gemäß Anspruch 1, dadurch gekennzeichnet, daß darin

$R^1$ und $R^2$ unabhängig voneinander für geradkettiges oder verzweigtes Alkyl mit 1 bis 6 Kohlenstoffatomen, geradkettiges oder verzweigtes Alkenyl und Alkinyl mit jeweils 2 bis 6 Kohlenstoffatomen, für gegebenenfalls ein- bis dreifach, gleich oder verschieden durch Methyl oder Ethyl substituiertes Cycloalkyl bzw. Cycloalkenyl mit 5 bis 7 Kohlenstoffatomen, für jeweils geradkettiges oder verzweigtes Alkoxy, Alkoxyalkylenoxy oder Alkoxyalkyl mit jeweils 1 bis 6 Kohlenstoffatomen in den einzelnen Alkylteilen, für Halogenalkyl mit 1 bis 6 Kohlenstoffatomen und 1 bis 5 Halogenatomen (insbesondere Fluor, Brom und Chlor), für Benzyl sowie für gegebenenfalls ein- bis dreifach, gleich oder verschieden substituiertes Phenyl stehen (wobei als Substituenten besonders bevorzugt sind: Methyl, Ethyl, Methoxy, Methylthio, Trifluormethyl, Trifluormethoxy, Trifluormethylthio, Fluor, Chlor oder Nitro) oder

$R^1$ und $R^2$ zusammen mit dem Stickstoffatom, an das sie gebunden sind, für einen gegebenenfalls ein- bis dreifach, gleich oder verschieden substituierten Heterocyclus der Formel

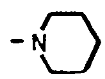 ; 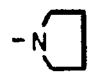 ; 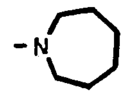 ; 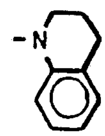

stehen, wobei als Substituenten besonders bevorzugt sind: Methyl, Ethyl und Phenyl.

3. Verfahren zur Herstellung von 5-Chlor-1,3,4-thiadiazol-2-yloxy-acetamiden der allgemeinen Formel (I)

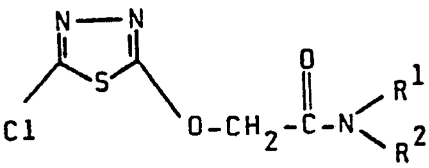
(I)

gemäß Anspruch 1, dadurch gekennzeichnet, daß man 2-Alkylsulfonyl-5-chlor-1,3,4-thiadiazole der Formel (II)

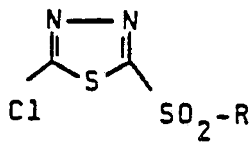
(II)

in welcher

R für Alkyl steht, mit Glykolsäureamiden der Formel (III),

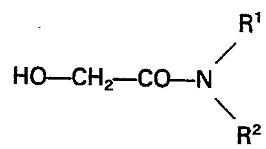
(III)

in welcher

$R^1$ und $R^2$ die oben angegebene Bedeutung haben, gegebenenfalls in Gegenwart eines Verdünnungsmittels und gegebenenfalls in Gegenwart eines Säurebindemittels sowie eines Katalysators umsetzt.

4. Herbizide Mittel, gekennzeichnet durch einen Gehalt an mindestens einem 5-Chlor-1,3,4-thiadiazol-2-yloxy-acetamid der allgemeinen Formel (I) gemäß Anspruch 1.

5. Verwendung von 5-Chlor-1,3,4-thiadiazol-2-yloxy-acetamiden der allgemeinen Formel (I) gemäß Anspruch 1 zur Bekämpfung von unerwünschtem Pflanzenwachstum.

6. Verfahren zur Herstellung von herbiziden Mitteln, dadurch gekennzeichnet, daß man 5-Chlor-1,3,4-thiadiazol-2-yloxy-acetamide der allgemeinen Formel (I) gemäß Anspruch 1 mit Streckmitteln und/oder oberflächenaktiven Mitteln vermischt.

**Revendications**

1. 5-chloro-1,3,4-thiadiazol-2-yloxy-acétamides de formule générale (I)

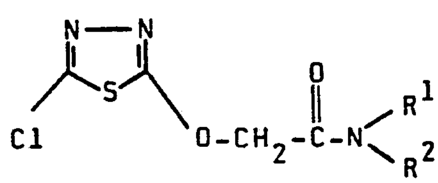
(I)

dans laquelle

$R^1$ et $R^2$ représentent indépendamment l'un de l'autre un groupe alkyle à chaîne linéaire ou ramifiée avec 1 à 8 atomes de carbone, un groupe alcényle ou alcynyle à chaîne linéaire ou ramifiée avec chaque fois 2 à 8 atomes de carbone, cycloalkyle ou cycloalcényle avec chaque fois 3 à 7 atomes de carbone, éventuellement substitué une fois ou plusieurs fois de façon identique ou différente (les substituants entrant particulièrement en considération étant les restes alkyle avec 1 à 4 atomes de carbone), un groupe alcoxy, alcoxyalkylèneoxy ou alcoxyalkyle, chaque fois à chaîne linéaire ou ramifiée et chaque fois avec 1 à 8 atomes de carbone dans les parties alkyle ou alkylène individuelles, un groupe halogénoalkyle avec 1 à 8 atomes de carbone et 1 à 5 atomes d'halogène, un groupe aralkyle avec 6 à 10 atomes de carbone dans la partie aryle et 1 à 2 atomes de carbone dans la partie alkyle, ainsi qu'un groupe aryle avec 6 à 10 atomes de carbone, éventuellement substitué une fois ou plusieurs fois de façon identique ou différente (des substituants entrant en considération étant: un halogène, un groupe alkyle, alcoxy ou alkylthio, chaque fois à chaîne linéaire ou ramifiée et chaque fois avec 1 à 4 atomes de carbone, halogénoalkyle, halogénoalcoxy et halogénoalkylthio avec chaque fois 1 à 2 atomes de carbone et 1 à 5 atomes d'halogène, ainsi que le groupe nitro),

$R^1$ et $R^2$ représentent, ensemble avec l'atome d'azote auquel ils sont liés, un hétérocycle avec 5 à 7 chaînons, saturé ou insaturé, éventuellement substitué une fois ou plusieurs fois de façon identique ou différente (les substituants entrant en considération étant un groupe alkyle avec 1 à 6 atomes de carbone, à chaîne linéaire ou ramifiée, également sous forme de système cyclique condensé, un groupe aryle avec 6 à 10 atomes de carbone, également sous forme de système cyclique condensé ou des groupes dioxyalkylène avec 2 à 3 atomes de carbone).

2. Composés de formule générale (I) selon la revendication 1, caractérisé en ce que, dans cette formule:

$R^1$ et $R^2$ représentent indépendamment l'un de l'autre un groupe alkyle à chaîne linéaire ou ramifiée avec 1 à 6 atomes de carbone, un groupe alcényle ou alcynyle à chaîne linéaire ou ramifiée avec chaque fois 2 à 6 atomes de carbone, cycloalkyle ou cycloalcényle avec 5 à 7 atomes de carbone, éventuellement substitué une à trois fois de façon identique ou différente par un groupe méthyle ou éthyle, un groupe alcoxy, alcoxyalkylèneoxy ou alcoxyalkyle, chaque fois à chaîne linéaire ou ramifiée et chaque fois avec 1 à 6 atomes de carbone dans les parties alkyle individuelles, un groupe halogénoalkyle avec 1 à 6 atomes de carbone et 1 à 5 atomes d'halogène (en particulier le fluor, le brome et le chlore), un groupe benzyle ainsi qu'un groupe phényle éventuellement substitué une à trois fois de façon identique ou différente, les substituants particulièrement préféres étant: méthyle, éthyle, méthoxy, méthylthio, trifluorométhyle, trifluorométhoxy, trifluorométhylthio, le fluor, le chlore ou un groupe nitro; ou

$R^1$ et $R^2$ représentent, ensemble avec l'atome d'azote auquel ils sont liés, un hétérocycle de formule

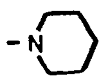 ; 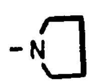 ; 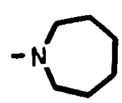 ; 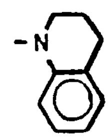

éventuellement substitué une à trois fois de façon identique ou différente, les substituants particulièrement préféres étant: méthyle, éthyle et phényle.

3. Procédé pour la fabrication de 5-chloro-1,3,4-thiadiazol-2-yloxy-acétamides de formule générale (I)

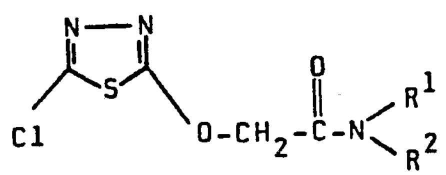

(I)

selon la revendication 1, caractérisé en ce que l'on fait réagir des 2-alkylsulfonyl-5-chloro-1,3,4-thiadiazols de formule (II)

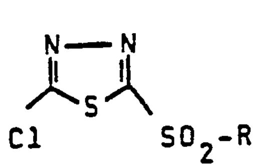

(II)

dans laquelle

R représente alkyle, avec des amides d'acides glycoliques de formule (III)

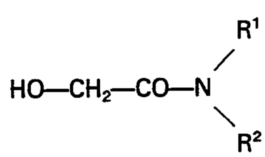

(III)

dans laquelle

$R^1$ et $R^2$ ont les significations décrites ci-dessus, éventuellement en présence d'un agent de dilution et éventuellement en présence d'un fixateur d'acide ainsi que d'un catalyseur.

4. Agent herbicide, caractérisé en ce qu'il contient au moins un 5-chloro-1,3,4-thiadiazol-2-yloxy-acétamide de formule générale (I) selon la revendication 1.

5. Utilisation des 5-chloro-1,3,4-thiadiazol-2-yloxy-acétamides de formule générale (I) selon la revendication 1 pour la lutte contre les croissances indésirables de plantes.

6. Procédé pour la fabrication d'agents herbicides, caractérisé en ce qu'on mélange des 5-chloro-1,3,4-thiadiazol-2-yloxy-acétamides de formule générale (I) selon la revendication 1 avec des agents de dilution et/ou des agents tensio-actifs.

**Claims**

1. 5-Chloro-1,3,4-thiadiazol-2-yloxy-acetamides of the general formula (I)

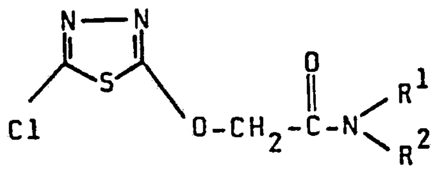

(I)

in which

$R^1$ and $R^2$ independently of one another represent straight-chain or branched alkyl with 1 to 8 carbon atoms, straight-chain or branched alkenyl and alkinyl each with 2 to 8 carbon atoms, cycloalkyl or cycloalkenyl, each with 3 to 7 carbon atoms, which optionally have one or more identical or different substituents (possible substituents being, in particular, alkyl radicals with 1 to 4 carbon atoms), or represent alkoxy, alkoxyalkyleneoxy or alkoxyalkyl, each of which is straight-chain or branched and has 1 to 8 carbon atoms in the individual alkyl or alkylene moieties, or represent halogenoalkyl with 1 to 8 carbon atoms and 1 to 5 halogen atoms or represent aralkyl with 6 to 10 carbon atoms in the aryl moiety and 1 to 2 carbon atoms in the alkyl moiety, as well as aryl with 6 to 10 carbon atoms which optionally has one or more identical or different substituents (possible substituents being: halogen, straight-chain or branched alkyl, alkoxy or alkylthio each with 1 to 4 carbon atoms, halogenoalkyl, halogenoalkoxy and halogenoalkylthio each with 1 to 2 carbon atoms and 1 to 5 halogen atoms, as well as nitro), or $R^1$ and $R^2$ together with the nitrogen atom to which they are bonded represent a saturated or unsaturated 5- to 7-membered heterocyclic ring which optionally has one or more identical or different substituents (possible substituents being: straight-chain or branched alkyl with 1 to 6 carbon atoms, also in the form of a fused ring system, aryl with 6 to 10 carbon atoms, also in the form of a fused ring system, or dioxyalkylene with 2 to 3 carbon atoms).

2. Compounds of the general formula (I) according to Claim 1, characterized in that, in this formula,

$R^1$ and $R^2$ independently of one another represent straight-chain or branched alkyl with 1 to 6 carbon atoms, straight-chain or branched alkenyl and alkinyl each with 2 to 6 carbon atoms, cycloalkyl or cycloalkenyl which have 5 to 7 carbon atoms and which are optionally mono- to tri-substituted by methyl or ethyl, the substituents being identical or different, or represent alkoxy, alkoxyalkyleneoxy or alkoxyalkyl, each of which is straight-chain or branched and has 1 to 6 carbon atoms in the individual alkyl moieties, or represent halogenoalkyl with 1 to 6 carbon atoms and 1 to 5 halogen atoms (especially fluorine, bromine and chlorine), or represent benzyl as well as phenyl which optionally has 1 to 3 identical or different substituents (particularly preferred substituents being: methyl, ethyl, methoxy, methylthio, trifluoromethyl, trifluoromethoxy, trifluoromethylthio, fluorine, chlorine or nitro) or

$R^1$ and $R^2$ together with the nitrogen atom to which they are bonded represent a heterocyclic ring of the formula

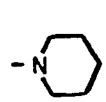 ; 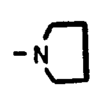 ; 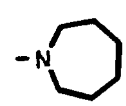 ; 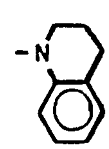

which optionally has one to three identical or different substituents, particularly preferred substituents being: methyl, ethyl and phenyl.

3. Process for the preparation of 5-chloro-1,3,4-thiadiazol-2-yloxy-acetamides of the general formula (I)

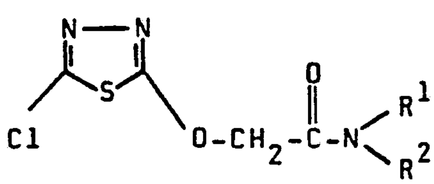

(I)

according to Claim 1, characterized in that 2-alkylsulphonyl-5-chloro-1,3,4-thiadiazoles of the formula (II)

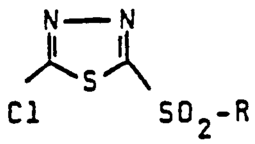

(II)

in which

R represents alkyl, are reacted with glycol amides of the formula (III)

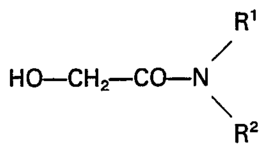

(III)

in which

$R^1$ and $R^2$ have the abovementioned meaning, if appropriate in the presence of a diluent, and, if appropriate, in the presence of an acid acceptor as well as a catalyst.

4. Herbicidal agents, characterized in that they contain at least one 5-chloro-1,3,4-thiadiazol-2-yloxy-acetamide of the general formula (I) according to Claim 1.

5. Use of 5-chloro-1,3,4-thiadiazol-2-yloxy-acetamides of the general formula (I) according to Claim 1 for combating undesired plant growth.

6. Process for the preparation of herbicidal agents, characterized in that 5-chloro-1,3,4-thiadiazol-2-yloxy-acetamides of the general formula (I) according to Claim 1 are mixed with extenders and/or surface-active agents.